# EUROPÄISCHE PATENTSCHRIFT

(11) **EP 3 626 195 B1**
(45) Veröffentlichungstag und Bekanntmachung des Hinweises auf die Patenterteilung: **27.01.2021**
(21) Anmeldenummer: 19197444.3
(22) Anmeldetag: 16.09.2019
(51) Int. Cl.: A61B 18/14, A61B 18/00, A61B 18/12

(54) **BIPOLARE HF-CHIRURGISCHE EFFEKTOREN PINZETTEN- ODER ZANGENARTIGER HF-CHIRURGISCHER DISSEKTIONSINSTRUMENTE**
BIPOLAR HF SURGICAL EFFECTORS OF FORCEP OR GRIPPER STYLE HF SURGICAL DISSECTION INSTRUMENTS
EFFECTEURS CHIRURGICAUX HF BIPOLAIRES DES INSTRUMENTS DE DISSECTION CHIRURGICALE HF SOUS LA FORME D'UNE PINCE OU D'UNE PINCETTE

(30) Priorität: 17.09.2018 EP 18194697
(43) Veröffentlichungstag der Anmeldung: 25.03.2020
(73) Patentinhaber: Farin, Günter, 72070 Tübingen (DE)
(72) Erfinder: Farin, Günter, 72070 Tübingen (DE)
(74) Vertreter: Lohr, Jöstingmeier & Partner

(56) Entgegenhaltungen:
- EP-A2- 2 044 900
- DE-T2- 69 823 437
- US-A1- 2016 066 980
- US-A1- 2017 164 997

## Beschreibung

### Technisches Gebiet

Die Erfindung betrifft Effektoren pinzetten- oder zangenartiger bipolarer HF-chirurgischer Dissektionsinstrumente, mit welchen biologische Gewebe, insbesondere Blutgefäße und Blutgefäße enthaltende Gewebe blutungsfrei durchtrennt bzw. geschnitten werden können.

### Stand der Technik

Die HF-chirurgische Gewebetrennung hat im Vergleich zur mechanischen Gewebetrennung per Skalpell oder Schere u. a. den Vorteil, dass auch Sehnen und faserhaltige Gewebe gut durchtrennt bzw. geschnitten werden können und insbesondere, dass Blutgefäße schnittsynchron durch thermische Koagulation und Gewebeschrumpfung infolge Desikkation (Trocknung) geschlossen und so Blutungen vermieden werden können. Großlumige Blutgefäße müssen für einen sicheren Gefäßverschluss während der Koagulation / Desikkation platt gedrückt bzw. zusammengequetscht werden. Hierfür stehen verschiedene HF-chirurgische Instrumente, insbesondere bipolare HF-chirurgische Dissektionspinzetten und bipolare HF-chirurgische Dissektionszangen in verschiedenen Ausführungsformen zur Verfügung.

Eine bipolare HF-chirurgische Dissektionspinzette für die offene Chirurgie ist beispielsweise aus dem europäischen Patent EP 1 778 112 bekannt. Bipolare HF-chirurgische Dissektionszangen für die offene Chirurgie sowie HF-chirurgische Zangen und Pinzetten für die minimal invasive Chirurgie sind beispielsweise aus der amerikanischen Patentschrift US 6,273,887 bekannt. Weitere HF-chirurgischen Zangen sind aus den Dokumenten EP2044900, US2016/066980, DE69823437 und US2017/164997 bekannt. Die zweiteilige Form des Anspruchs 1 basiert auf EP2044900.

Fast alle bipolare HF-chirurgische Dissektionspinzetten und Dissektionszangen umfassen an ihren distalen Enden einen Effektor, auch Maul genannt, mit in der Regel zwei elektrisch voneinander isolierten Effektorelementen (auch Maulteile genannt), die zum mechanischen Greifen und/oder Quetschen sowie zum bipolaren HF-chirurgischen Koagulieren und/oder Trennen (Schneiden) biologischer Gewebe aufeinander zu (auch schießen genannt) und voneinander weg (auch öffnen genannt) bewegt werden können, und einen Aktor (auch Griff genannt) an ihrem proximalen Ende, mit welchem der Effektor manuell geschlossen und geöffnet werden kann.

Bezüglich der konstruktiven Gestaltung und Funktion der Effektoren sind viele verschiedene Lösungen vorgeschlagen worden, wovon einige technisch sehr kompliziert und aufwendig produzierbar und/oder nur relativ wenig klinisch praktikabel sind. Man unterscheidet bipolare HF-chirurgische Effektoren mit nur zwei Elektroden, die sowohl zum Greifen und/oder Quetschen als auch zum Koagulieren und / oder Gewebetrennen (Schneiden) angewendet werden können, von bipolaren HF-chirurgischen Effektoren mit mehr als zwei Elektroden, wobei Elektrodenpaare zum bipolaren Koagulieren von Elektrodenpaare zum bipolaren Trennen von Geweben voneinander getrennt angeordnet sind.

Ein besonderes Merkmal bipolarer HF-chirurgischer Effektoren vorbekannter HF-chirurgischer Dissektionspinzetten und Dissektionszangen sind Vorrichtungen zur Vermeidung elektrischer Kurzschlüssen zwischen den gegenpoligen Elektrodenpaaren, wenn diese beim Schließen des Effektors aufeinander zubewegt werden. Das ist insbesondere im Schneidemodus relevant, weil hierbei das zwischen den gegenpoligen Elektroden zum Durchtrennen befindliche Gewebe weggebrannt wird. Würde ein Kurzschluss entstehen, so kann dieser Vorgang nicht fortgesetzt werden. Das soll bei vorbekannten Effektoren durch die o. g. Vorrichtung zur Vermeidung elektrischer Kurzschlüsse vermieden werden. Eine plausible Begründung für dieses Merkmal, außer dass hierdurch ein Kurzschluss vermieden werden soll, wird nicht genannt. Ein plausibler Grund hierfür ist vermutlich das Risiko, das die Effektoren während einer HF-chirurgischen Gewebetrennung, im sogenannten Schneidemodus, einander berühren könnten, bevor die beabsichtigte Gewebetrennung vollendet ist, sodass wegen des durch die Berührung verursachten Kurzschlusses die Gewebetrennung nicht vollendet werden kann. Hierdurch wird allerdings ein anderes Problem verursacht.

Bekanntlich wird die HF-chirurgische Gewebetrennung - auch Schneiden genannt - durch heiße elektrische Lichtbogen verursacht, die das von ihnen getroffene Gewebe wegbrennen, d. h. in Dampf und Rauch auflösen. Elektrische Lichtbogen zwischen einer Elektrode und Gewebe können bekanntlich nur da und dann entstehen, wo und wenn eine zum HF-chirurgischen Schneiden geeignete Elektrode das zu schneidende Gewebe nicht direkt und elektrisch leitfähig berührt, sodass bei ausreichend hoher Amplitude der elektrischen Feldstärke zwischen Schneidelektrode und Gewebe die lonisationsfeldstärke des dazwischen befindlichen Gases erreicht und heiße elektrische Lichtbogen entstehen. Die elektrische Feldstärke ist hierbei bekanntlich direkt proportional der elektrischen Spannung und reziprok proportional dem Abstand zwischen Elektrode und Gewebe. Die o. g. Vorrichtung zur Vermeidung des Kurzschlusses zwischen gegenpoligen Elektroden bei Effektoren zum Beispiel o. g. bekannter bipolarer Dissektionspinzetten und Dissektionszangen durch Einfügung einer Isolationsstrecke zwischen den gegenpoligen Elektroden verursacht einen Konflikt mit der zum HF-chirurgischen Schneiden erforderlichen Amplitude der HF-Spannung als Voraussetzung zur Erreichung der Ionisations-Feldstärke in der Isolationsstrecke. Je größer der Isolationsabstand, desto höher muss die Amplitude der HF-Spannung zur Erreichung der lonisationsfeldstärke sein. Die Isolationsstrecke zur Vermeidung eines Kurzschlusses zwischen gegenpoligen Elektrode muss hierbei berücksichtigt werden, also entsprechend höher sein als ohne Isolationsstrecke. Bei bekannten bipolaren Effektoren mit nur zwei gegenpoligen Elektroden, bei welchen nur eine der beiden Elektroden am selben Gewebe und an derselben Stelle des Gewebes sowohl zum Koagulieren als auch zum Schneiden angewendet wird, können während der Schneidphase elektrische Lichtbogen auch da entstehen, wo Gewebe koaguliert, aber nicht weggebrannt werden darf.

Darüber hinaus besteht bei bekannten bipolaren Effektoren mit nur zwei Elektroden, zwischen welchen eine Isolationsstrecke zur Vermeidung eines Kurzschlusses eingefügt ist das Problem, dass nach Vollendung des HF-chirurgischen Schnitts zwar dort keine elektrischen Lichtbogen mehr entstehen, wo der Schnitt erfolgte, aber dann, wie bereits oben bemerkt, zu Gewebe entstehen, wo nicht geschnitten, sondern nur koaguliert werden darf. Da Chirurgen nicht erkennen können, ob elektrische Lichtbogen noch das zu schneidende oder bereits das zu koagulierende Gewebe erreichen, wird mehr Gewebe thermisch geschädigt als erforderlich oder mehr als erlaubt.

### Darstellung der Erfindung

Der Erfindung liegt die Aufgabe zugrunde, einen bipolaren HF-chirurgischen Effektor für Dissektionspinzetten und Dissektionszangen oder dergleichen medizinische Instrumente zur Verfügung zu stellen, bei welchen die oben genannten Probleme nicht vorhanden sind.

Entgegen dem Stand der Technik und der vorherrschenden Meinung der Fachleute auf diesem Gebiet wird ein bipolarer HF-chirurgischer Effektor mit nur zwei Elektroden vorgeschlagen, bei welchem ein elektrischer Kurzschluss während des HF-chirurgischen Schneidemodus nicht nur geduldet, sondern sogar beabsichtigt ist. Kurzschlüsse zwischen den zwei gegenpoligen Elektroden können im Schneidemodus nicht nur nach Vollendung des beabsichtigten Schnitts, sondern partiell auch vorher entstehen. Passiert das, dann können während der Existenz des partiellen Kurzschlusses und auch nach Vollendung des Schnitts keine unerwünschten oder gar schädlichen elektrische Lichtbogen gegen das Gewebe entstehen.

Vorzugsweise umfasst ein erfindungsgemäßer bipolarer HF-chirurgischer Effektor, der Bestandteil sowohl von Dissektionspinzetten als auch von Dissektionszangen, endoskopischen Instrumenten oder dergleichen medizinischen Instrumenten sein kann, prinzipiell ähnlich wie Effektoren mechanischer Ambossscheren, zwei Effektorelemente. Ein erstes Effektorelement, welches eine keilartige Form haben kann, umfasst einen elektrisch leitfähigen Schneidgrat und einseitig oder beidseitig und bevorzugt längs am Schneidgrat anschließende elektrisch leitfähige Flanken. Ein zweites Effektorelement kann eine Schneidbahn umfassen, wobei mit dem Unterschied zu einer mechanischen Ambossschere der Schneidgrat des Effektors bevorzugt nicht mechanisch scharf, sondern vorzugsweise stumpf sein soll. Bevorzugt derart, dass das damit zu schneidende Gewebe während der Koagulationsphase im Sinne eines mechanothermischen Gefäßverschlusses mit dem Schneidgrat gegen die Schneidbahn gequetscht werden kann. Dadurch kann außerdem, wie bereits oben bemerkt, der Abstand zwischen Schneidgrat und Schneidbahn zugunsten einer möglichst hohen elektrischen Feldstärke im zusammengequetschten Gewebe bei möglichst geringer Amplitude der HF-Spannung minimiert werden.

Der Schneidgrat und die Schneidbahn des erfindungsgemäßen Effektors umfassen jeweils ein distales Ende und ein proximales Ende und haben dazwischen jeweils eine vorgegebene Länge. Schneidgrat und Schneidbahn sind beim erfindungsgemäßen Effektor vorzugsweise so gestaltet, dass jede Stelle bzw. jeder Punkt des Schneidgrats während des Schneidens die Schneidbahn bevorzugt an einem Punkt, direkt berühren kann. Die Position des Punktes der Berührung kann sich während des Schneidvorganges ändern. Hierzu ist der Schneidgrat und/oder die Schneidbahn vorzugsweise so gestaltet, dass der Schneidgrat des Effektors beispielsweise wie der Schneidgrat eines Wiegemessers kraftschlüssig auf der Schneidbahn beispielsweise wiegend vor- und zurückbewegt und / oder vor- und zurückgerollt werden kann. Hierzu ist der Schneidgrat und/oder die Schneidbahn in Längsrichtung vorzugsweise konvex gebogen. Um diese insbesondere (wiegende) rollende Bewegung zwischen Schneidgrat und Schneidbahn zu ermöglichen, umfasst vorzugsweise mindestens einer der beiden Effektorelemente möglichst nahe am proximalen Ende des Schneidgrats und/oder am proximalen Ende der Schneidbahn ein biegeelastisches und/oder federelastisches Element oder Biegeelement, das bei zangenartigen Instrumenten vorzugsweise distal vor deren Drehgelenk, und bei pinzettenartigen Instrumenten vorzugsweise am distalen Ende ihrer Branchen angeordnet ist, wobei hier die Branchen als Gelenk wirken. Diese Eigenschaft des federelastischen Biegeelements könnte auch in mindestens einer der beiden Pinzettenbranchen einer Pinzette vorhanden sein, sodass hier kein separates federelastisches Element erforderlich sein könnte. Bevorzugt erfolgt eine Führung der Effektorelemente durch das Gelenk (Drehgelenk bei zangenartigen Instrumenten, Branchen bei Pinzetten) innerhalb einer Ebene. Daraus ergibt sich bevorzugt eine Bewegung innerhalb einer Ebene, in welcher auch bevorzugt die Effektorelemente bzw. deren Schneidgrat oder Schneidbahn angeordnet sind.

Bei den hier beschriebenen Instrumenten handelt es sich um bipolare Instrumente. Daher sind in einem geöffneten Zustand die beiden Effektorelemente elektrisch voneinander isoliert. Diese können dann an die zwei Pole eines bipolaren HF-Chirurgiegenerators angeschlossen werden. Erst durch die Betätigung des Instruments nähern sich die beiden Effektorelemente derart an, dass sie einander berühren können und einen geschlossenen Zustand annehmen. In diesem geschlossenen Zustand können dann die Effektorelemente weiter zwischen mehreren Positionen bewegt werden, sodass die hier beschriebene Bewegung entsteht.

Sind federelastische und vorzugsweise identische Biegeelemente an den proximalen Enden der beiden Effektorelemente vorhanden, entsteht bei kraftschlüssiger Annäherung von Schneidgrat und Schneidbahn ein kraftschlüssiges Abrollen aufeinander. Ist ein federelastisches Biegeelement nur an einem Effektorelement vorhanden, oder sind die federelastischen Biegeelemente unsymmetrisch ausgeführt, dann kann bei kraftschlüssiger Annäherung von Schneidgrat und Schneidbahn ein zusätzlich zum kraftschlüssigen Abrollen auch ein Gleiten aufeinander entstehen. Das Gleiten aufeinander kann die Kontakte zwischen Schneidgrat und Schneidbahn von dort angebranntem Gewebe reinigen.

Am proximalen Ende der beiden Effektorelemente oder federelastischen Biegeelemente kann ein pinzettenartiger oder zangenartiger Aktuator (auch Griff genannt) angeschlossen werden, mit welchen die beiden Effektorelemente in an sich bekannter Weise kinematisch aufeinander zu (auch schließen genannt) und voneinander weg (auch öffnen genannt) werden können. Derartige Aktuatoren für bipolare Pinzetten sowie bipolare Zangen inklusive bipolare Anschluss-Stecker und -Kabel zum Anschluss an Hochfrequenz-Chirurgiegeräte sind aus dem Stand der Technik bekannt und werden deswegen hier nicht beschrieben.

Elektrische Kurzschlüsse zwischen Schneidgrat und Schneidbahn während des Schneidens können automatisch identifiziert und dem Chirurgen z. B. akustisch und/oder optisch signalisiert werden. Hierzu ist beispielsweise eine Vorrichtung geeignet, welche die Amplitude der HF-Spannung zwischen Schneidgrat und Schneidbahn, während der Schneidphase überwacht und bei Kurzschluss, also Amplitude der HF-Spannung nahe Null Volt, o. g. Signale erzeugt. Mit einer derartigen Vorrichtung kann auch kontrolliert werden, ob ein Schnitt über die gesamte Länge des Schneidgrats vollständig oder nicht vollständig ist, und wenn nicht, wo nicht. Letzteres ist insbesondere bei Effektoren mit konvexem Schneidgrat und / oder konvexer Schneidbahn möglich, bei welchen eine elektrisch leitfähige Berührung (elektrischer Kurzschluss) längs des Schneidgrats und der Schneidbahn punktuell entsteht.

Der erfindungsgemäße Effektor kann sowohl nur zum Schließen von Blutgefäßen insbesondere mit hierfür optimal geeigneter Amplitude der HF-Spannung kleiner 200 Vp, als auch zum Gewebetrennen mit oder ohne schnittsynchronem Gefäßverschluss sowie Gewebetrennen nach vorherigem mechanothermische(n) m (quetschen und gleichzeitig koagulieren) Gefäßverschluss angewendet werden.

Je nach Anwendungsbereich des erfindungsgemäßen Effektors sind verschiedene anwendungs-spezifische konstruktive und/oder ergonomische Variationen und Ausgestaltungen des oben allgemein beschriebenen bipolaren HF-chirurgischen Effektors möglich.

Der Effektor kann in axialer Richtung gerade, gebogen und/oder abgewinkelt sein.

Die zum Koagulieren nützlichen Flanken seitlich des Schneidgrats können verschieden steil und/oder hoch sein. Je flacher und/oder höher, desto größer die hiermit erzeugbare Koagulationszone lateral des Schnitts.

Der Schneidgrat und dessen Flanken sowie die Schneidbahn bestehen vorzugsweise aus einem elektrisch leitfähigen Material. Die Koagulationsflanken seitlich des Schneidgrats und / oder die Koagulationsböschungen der Schneidbahn können oder sind mit einem Material beschichtet, an dem koaguliertes Gewebe selten oder gar nicht festklebt.

Erfindungsgemäße Effektoren können ausreichend klein und/oder schlank hergestellt werden, sodass sie nicht nur an pinzettenartigen und zangenartigen Instrumenten für die offene Chirurgie, sondern auch an Instrumenten für die sogenannte (M) minimal invasive Chirurgie (MIC) angewendet werden können.

Ein bipolarer HF-chirurgischer Effektor umfasst ein erstes Effektorelement sowie ein zweites Effektorelement. Das erste Effektorelement kann mit dem zweiten Effektorelement durch ein Gelenk verbunden sein. Das Gelenk kann ein Drehgelenk bei zangenartigen Instrumenten oder auch Branchen bei Pinzetten umfassen. Das Gelenk kann eine Bewegung des ersten Effektorelements gegenüber dem zweiten Effektorelement ausschließlich in einer ersten Ebene zulassen. Der Effektor kann zwischen einem geöffneten Zustand und einem geschlossenen Zustand und im geschlossenen Zustand zwischen mehreren Positionen bewegt werden. Ein federelastisches Element kann zwischen dem Gelenk und wenigstens einem der Effektor-Elemente angeordnet sein. Das erste Effektorelement kann einen elektrisch leitfähigen Schneidgrat in der ersten Ebene mit bevorzugt wenigstens einer längs des Schneidgrats angeordneten elektrisch leitfähigen Koagulationsflanke umfassen. Es können auch zwei elektrisch leitfähige Koagulationsflanken beidseits des Schneidgrats angeordnet sein. Das zweite Effektorelement kann eine gegenüber dem Schneidgrat angeordnete elektrisch leitfähige Schneidbahn oder einen zweiten Schneidgrat umfassen. In einem geöffneten Zustand des Effektors ist der Schneidgrat gegen die Schneidbahn oder den zweiten Schneidgrat elektrisch isoliert. In einem geschlossenen Zustand kann ein elektrischer Kurzschluss zwischen Schneidgrat und Schneidbahn bestehen.

Der Schneidgrat und/oder die Schneidbahn können eine konvexe Krümmung aufweisen.

Der HF-chirurgische Effektor kann derart konfiguriert sein, dass im geschlossenen Zustand des Effektors der Schneidgrat die Schneidbahn in genau einem Punkt berührt, der sich während der Bewegung des Effektors zwischen wenigstens zwei Positionen des geschlossenen Zustands entlang des Schneidgrats und der Schneidbahn fortbewegt.

Der HF-chirurgische Effektor kann derart konfiguriert sein, dass durch eine kontinuierliche Bewegung des Effektors im geschlossenen Zustand eine kontinuierliche Bewegung des Punkts der Berührung bevorzugt ohne Unterbrechung erfolgt und/oder bei einer Fortsetzung der Bewegung des Effektors von einem geöffneten Zustand in einen geschlossenen Zustand, im geschlossenen Zustand eine Bewegung des Punkts der Berührung in proximaler Richtung erfolgt.

Der HF-chirurgische Effektor kann derart konfiguriert sein, dass an dem Punkt der Berührung ein elektrischer Kurzschluss zwischen dem Schneidgrat und der Schneidbahn ist.

Der Schneidgrat kann an einer Seite eine elektrisch leitfähige Koagulationsflanke oder an zwei Seiten jeweils eine elektrisch leitfähige Koagulationsflanke umfassen.

Der Schneidgrat kann zwei unter einem Flankenwinkel zusammentreffende Flächen umfassen, wobei die zwischen den Flächen entstehende Kante zur Schneidbahn hin orientiert ist und der Flankenwinkel zwischen einer der Flächen und einer Fläche der Schneidbahn wahlweise kleiner 150°, kleiner als 110°, kleiner als 90°, kleiner als 60° ist.

Die Schneidbahn kann eine Führungsrille für den Schneidgrat (4a) des ersten Effektor-Elements oder einen zweiten Schneidgrat umfassen.

Die Oberflächen des Effektors und/oder der Komponenten des Gelenks können elektrisch nicht leitfähig sein und/oder mit einem elektrischen Isolationsmaterial beschichtet sein, wobei der Schneidgrat, die Koagulationsflanken und die Schneidbahn ausgenommen sein können und zumindest eine leitfähige Oberfläche umfassen können und/oder dass wenigstens eines von dem Schneidgrat, den Koagulationsflanken und der Schneidbahn mit einem Material beschichtet sein kann, das das Ankleben biologischer Gewebe reduziert oder verhindert.

Das federelastische Element kann mindestens einen elastischen Biegebereich derart umfassen, dass der HF-chirurgische Effektor von einem geöffneten Zustand in mehrere vorgegebene Positionen im geschlossenen Zustand bewegt werden kann, ohne dass eine plastische Verformung des federelastische Elements eintritt.

Der HF-chirurgische Effektor oder ein mit dem Effektor verbundenes Bauteil kann einen mechanischen Anschlag aufweisen, der die Bewegung des Effektors auf einen Bereich, der kleiner oder gleich dem elastischen Bereich des federelastischen Elements ist, einschränken kann.

Der Effektor kann Bestandteil einer Schere, einer Zange, einer Pinzette oder eines endoskopischen Instruments sein.

An einem proximalen Ende mindestens eines biegeelastischen Elements und/oder am proximalen Ende des ersten Effektorelements und/oder des Schneidgrats und/oder am proximalen Ende des Effektorelements und/oder der Schneidbahn und/oder am proximalen Ende des Gelenks kann ein Aktuator vorhanden sein, mit dem der Effektor bewegt und insbesondere geöffnet und geschlossen werden kann.

Das erste Effektorelement kann mit einem ersten Pol eines vorzugsweise bipolaren HF-Chirurgiegenerators verbunden und das zweite Effektorelement mit einem zweiten Pol des HF-Chirurgiegenerators verbunden sein.

Eine Schere, Zange, Pinzette oder ein endoskopisches Instrument kann einen HF-chirurgischen Effektor wie hier beschrieben, umfassen.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Fig. 1 zeigt das Prinzip eines Bipolaren HF-chirurgischen Effektor in Seitenansicht.
Fig. 2 zeigt das Prinzip eines Effektors im Querschnitt an einer Stelle, wo zwischen Schneidgrat und Schneidbahn ein elektrischer Kurzschluss vorhanden ist.
Fig. 3 zeigt einen Effektor mit einem Zugseil.
Fig. 4 zeigt einen Abrollvorgang.
Fig. 5 zeigt im Schnitt Schneidgrat und Schneidbahn.
Fig. 6 zeigt noch eine Pinzette mit einem Effektor.
Fig. 7 zeigt zwischen Effektorelementen eingeklemmtes Gewebe.
Fig. 8 zeigt das elektrische Feld bei aktiviertem HF-Generator.
Fig. 9 zeigt einen Lichtbogen zwischen den Effektorelementen.
Fig. 10 zeigt einen elektrischen Kurzschluss zwischen den Effektorelementen.

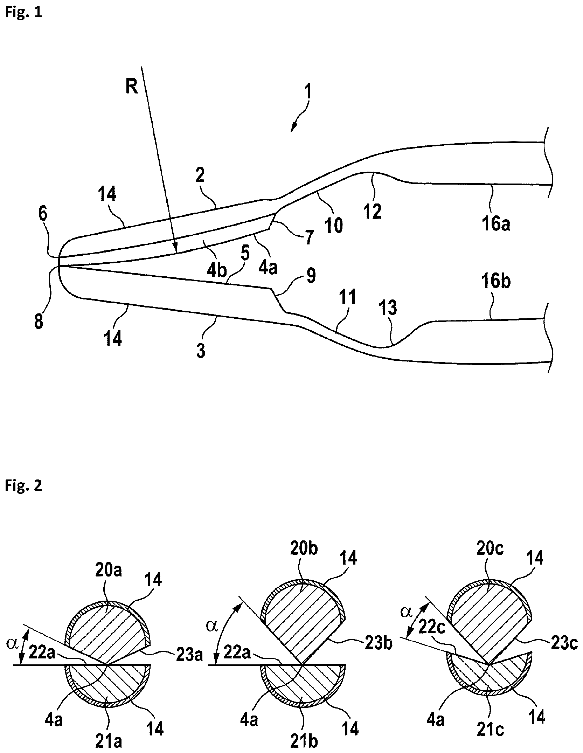

Die prinzipielle Konstruktion und Funktion eines bipolaren HF-chirurgischen Dissektionseffektors, im folgenden kurz Effektor genannt, entsprechend der Erfindung, der Bestandteil sowohl von bipolaren HF-chirurgischen Dissektionspinzetten als auch von bipolaren HF-chirurgischen Dissektionszangen sein kann, kann anhand von Figur 1 in Verbindung mit Figur 2 wie folgt beschrieben werden.

Der Effektor 1 umfasst zwei pinzetten- oder zangenartig aufeinander zu sowie voneinander weg bewegbare Effektorelemente 2, 3. Das erste Effektorelement 2 hat einen HF-chirurgischen Schneidgrat 4a und bevorzugt daran anschließend mindestens eine Koagulationsflanke 4b, die in Fig. 2 mit 23a, 23b und 23c gekennzeichnet sind. Auf der anderen Seite des Schneidgrats 4a ist, wie in Fig. 2 dargestellt, bevorzugt eine zweite Koagulationsflanke vorhanden. Das zweite Effektorelement 3 hat eine elektrisch leitfähige Schneidbahn 5, die in Figur 2 mit 22a bzw. 22c gekennzeichnet ist. Der Schneidgrat erstreckt sich vorzugsweise zwischen einem distalen Ende 6 und einem proximalen Ende 7. Die Schneidbahn erstreckt sich vorzugsweise zwischen einem distalen Ende 8 und einem proximalen Ende 9. Der Schneidgrat und/oder die Schneidbahn ist bzw. sind bevorzugt in Längsrichtung gerade oder haben in Längsrichtung einem Radius R. Bevorzugt haben der Schneidgrat (4a) und/oder die Schneidbahn (5) eine konvexe Krümmung. Konvex könnte hier bedeuten, dass die Mitte gegenüber dem Rand hervortritt. In Fig. 1 hat die Schneidbahn einen unendlich großen Radius, sie ist folglich gerade. Der Schneidgrat 4a inklusive der daran anschließenden Koagulationsflanken sowie die Schneidbahn inklusive der seitlich daran anschließenden Koagulationsböschungen 22a 22c sind mit einem Hochfrequenz-Chirurgiegerät verbindbar. Zwischen Schneidgrat und Schneidbahn könnte bevorzugt biologisches Gewebe pinzettenartig oder zangenartig gegriffen, gequetscht, bipolar HF-chirurgisch koaguliert und/oder bipolar HF-chirurgisch durchtrennt werden.

Zum Durchtrennen bzw. Schneiden von Gewebe müssen zwischen Schneidgrat und Gewebe und möglichst nur zwischen Schneidgrat und Gewebe ausreichend heiße elektrische Lichtbogen entstehen. Elektrische Lichtbogen entstehen primär da, wo die elektrische Ionisations-Feldstärke, auch Zündfeldstärke genannt, erreicht wird. Diese entstehen bei dem erfindungsgemäßen Effektor zwischen Schneidgrat und Schneidbahn, insbesondere in einer Dampfschicht, die während der Anschnittphase zwischen Schneidgrat und dem daran angrenzendem Gewebe bildet. Je kürzer der Abstand zwischen Schneidgrat 4a und Schneidbahn 5, desto höher ist die elektrische Feldstärke im Gewebe und desto schneller wird die Ionisations-Feldstärke in der während der Anschnittphase entstehende Dampfschicht zwischen Schneidgrat und Gewebe erreicht.

Wie in Figur 2 schematisch dargestellt, können die bevorzugt elektrisch leitfähigen, also nicht elektrisch isolierten Flanken 23a, 23b, 23c verschiedener Ausführungen von ersten Effektorelementen (20a, 20b, 20c) mehr oder weniger steil sein bzw. einen mehr oder weniger großen Winkel α relativ (zur Schneidbahn) zu den Koagulationsböschungen 22a, 22c haben. Je kleiner α, desto breiter wird die Koagulationszone links und / oder rechts des Schneidgrats und folglich auch effizienter der thermomechanische, also durch mechanische Quetschung und thermische Koagulation entstehende Gefäßverschluss. Die Flanken 23a, 23b, 23c und/oder die Böschungen 22a, 22c können aber auch teilweise elektrisch isoliert sein.

Die Effektorelemente sind an allen Stellen, die keinen elektrisch leitfähigen Kontakt zu Gewebe haben dürfen, mit einer elektrischen Isolation 14 beschichtet. Bevorzugt sind die Außenseiten elektrisch isoliert.

Der erfindungsgemäße Effektor ist dadurch gekennzeichnet, dass der Abstand zwischen Schneidgrat und Schneidbahn beliebig klein sein kann und darf, insbesondere bis zum elektrischen Kurzschluss zwischen Schneidgrat und Schneidbahn. Ein Kurzschluss zwischen Schneidgrat und Schneidbahn ist ein sicheres Indiz dafür, dass das Gewebe an der Stelle, wo der Kurzschluss besteht, bzw. vorhanden war, vollständig durchtrennt ist.

Sobald und solange zwischen Schneidgrat und Schneidbahn elektrischer Kurzschluss besteht, ist die Amplitude der elektrischen Spannung zwischen Koagulationsflanken 23a, 23b, 23c und Koagulationsböschungen 22a, 22c vernachlässigbar klein oder Null Volt. Das hat den Vorteil, dass während des Kurzschlusses keine unkontrollierbaren oder gar schädliche thermische Effekte in angrenzenden oder distanten Geweben entstehen.

Ein Kurzschluss zwischen Schneidgrat und Schneidbahn kann mit einer hierzu geeigneten Vorrichtung messtechnisch identifiziert und dem Chirurgen akustisch und oder visuell signalisiert werden. Der hier eingesetzte HF-Chirurgiegenerator ist bevorzugt kurzschlussfest und beginnt bevorzugt unmittelbar nach einer Beseitigung eines Kurzschlusses wieder mit der Leistungsabgabe.

Um sicherzustellen, dass das mit dem Effektor gegriffene und zu durchschneidende Gewebe vollständig durchschnitten wurde, ist es vorteilhaft, wenn der Schneidgrat und/oder die Schneidbahn in Längsrichtung derart gestaltet sind, dass der Schneidgrat die Schneidbahn nur partiell bzw. punktförmig berührt und diese Berührung durch kraftschlüssiges Abrollen des Schneidgrats auf der Schneidbahn und/oder der Schneidbahn auf dem Schneidgrat zwischen ihren distalen Enden 6, 8 und ihren proximalen Enden 7, 9 verlagert werden kann.

Um dieses kraftschlüssige Abrollen des Schneidgrats auf der Schneidbahn bzw. der Schneidbahn auf dem Schneidgrat auch dann praktizieren zu können, wenn der Effektor an einem pinzettenartigen oder zangenartigen Instrument angewendet wird, ist bevorzugt am proximalen Ende 7 des Schneidgrats 4a und/oder am proximalen Ende 9 der Schneidbahn 5 wenigstens ein federelastisches Biegeelement 10, 11 vorhanden, derart, dass bei Variation der Schließkraft und/oder einer Schließbewegung am proximalen Ende 12, 13 der federelastischen Elemente das kraftschlüssige Abrollen möglich ist. Bei Anwendung des erfindungsgemäßen Effektors an pinzettenartigen Instrumenten kann eventuell auf diskrete federelastische Biegeelemente verzichtet werden, wenn die Pinzettenbranchen diese Eigenschaft insbesondere im distalen Bereich mindestens einer Pinzettenbranche bereits haben.

Ist nur ein federelastisches Biegeelement entweder am Effektorelement 2 oder am Effektorelement 3 vorhanden, dann entsteht beim Schließen sowie beim Öffnen des Effektors zusätzlich eine Gleitbewegung zwischen Schneidgrat und Schneidbahn, was den Vorteil haben kann, dass am Schneidgrat und/oder auf der Schneidbahn angebackene Gewebereste weggerieben werden.

Selbstverständlich muss auch bei dem erfindungsgemäßen Effektor zu dessen praktischer Anwendung an dessen proximalem Ende ein Aktuator (auch Griff genannt) vorhanden sein, mit dem der Effektor geöffnet und geschlossen werden kann. Hierfür geeignete Aktuatoren in vielen verschiedenen pinzettenartigen und zangenartigen Ausführungsformen sind aus dem Stand der Technik bekannt.

Selbstverständlich müssen die beiden elektrischen Pole der elektrischen Spannung aus einer HF-Spannungsquelle sowohl dem Schneidgrat als auch der Schneidbahn zugeleitet werden. Auch hierzu gibt es verschiedene aus dem Stand der Technik bekannte und praktikable Lösungen.

Der erfindungsgemäße Effektor kann derart klein und/oder schlank sein, dass er auch an sogenannten Rohrschaft-Instrumenten für die minimal invasive Chirurgie (MIC) anwendbar ist.

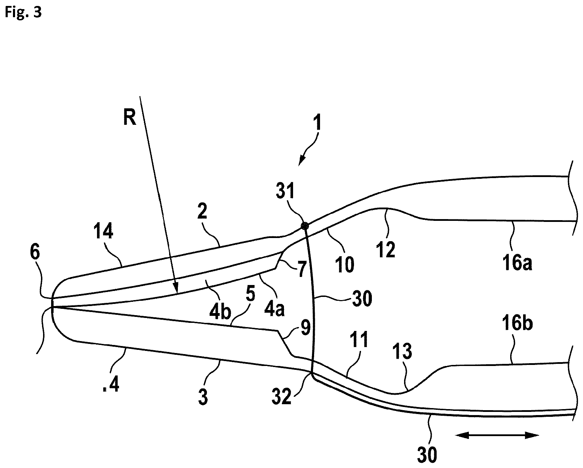

Zum Öffnen und Schließen des Effektors bei diesen Instrumenten ist es zweckmäßig, die Schließkraft mittels eines Zugseils auf den Effektor zu übertragen, wie es beispielsweise in Figur 3 schematisch dargestellt ist. Hierzu ist mindestens ein Zugseil 30 im Bereich des proximalen Endes 7 des Effektorelements 2 und/oder des Effektorelements 3, vorzugsweise im distalen Bereich des federelastischen Biegeelements 10 und/oder 11 an einem Punkt 31 befestigt und durch eine Führung, beispielsweise ein Loch (in der Seitenansicht von Figur 3 nicht direkt sichtbar) 32 in Richtung Aktuator umgelenkt. Das Loch 32 hat vorzugsweise keine scharfen Kanten, sodass das Zugseil ohne nennenswerte Reibung und Verletzung darin bewegt werden kann. Diese kinematische Einrichtung hat außerdem den Vorteil, dass die Zugkraft direkt und "direkt proportional" in Schließkraft umgewandelt wird. Das Zugseil kann innerhalb oder außerhalb des Rohrschafts geführt werden.

Bei Rohrschaft-Instrumenten kann eines der beiden Effektorelemente mit oder ohne biegeelastischem Element elektrisch leitfähig am elektrisch leitfähigen Rohrschaft befestigt sein, sodass der Rohrschaft als Leitung für den HF-Strom genutzt werden kann. Hierzu kann es zweckmäßig sein, dass der Rohrschaft außen mit einem elektrisch isolierenden Material beschichtet ist.

Um zu vermeiden, dass das Zugseil durch heiße elektrische Lichtbogen, die beim HF-chirurgischen Schneiden entstehen, thermisch beschädigt wird, ist es zweckmäßig den Befestigungspunkt 31 sowie das Loch 32 weiter nach proximal vom Schneidgrat zu distanzieren als die maximale Reichweite der elektrischen Lichtbogen ab dem proximalen Ende 7 des Schneidgrats 4a.

Der erfindungsgemäße Effektor kann sowohl in axialer Richtung des jeweiligen Instruments als auch seitlich abgebogen und/oder abgewinkelt realisiert und angewendet werden, was insbesondere bei Instrumenten für die MIC vorteilhaft ist, wo die Manipulation des Effektors durch den engen MIC-Zugang zum Operationsfeld bzw. Zielgewebe mit konventionellen oft schwierig oder gar nicht möglich ist.

Die Effektorelemente 2 und 3 sowie alle elektrische Spannung gegen Gewebe führen sind bevorzugt an Stellen, die nicht HF-chirurgisch genutzt werden, mit einem elektrisch isolierenden Material 14 beschichtet.

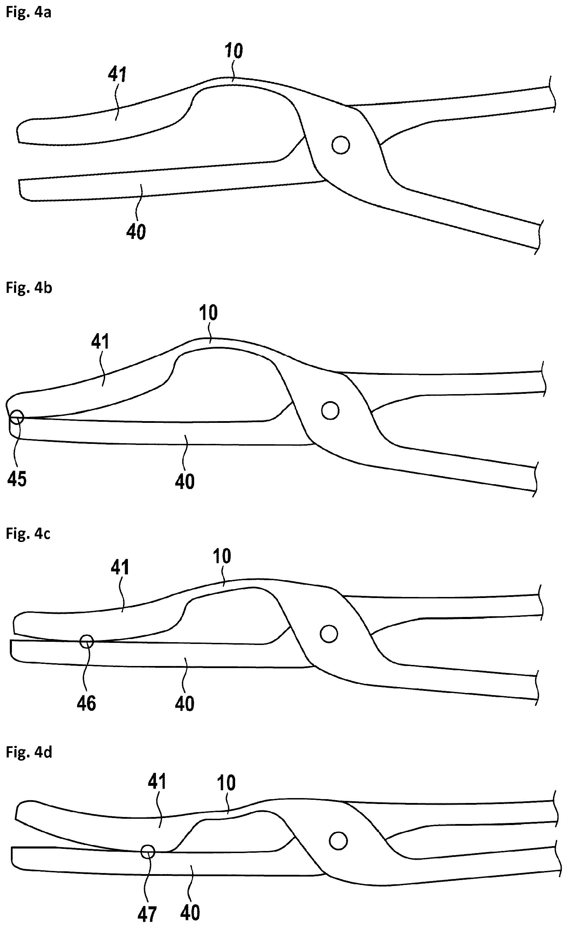

In der Figur 4a - 4d ist der Abrollvorgang des Schneidgrats 41 auf der Schneidbahn 40 am Beispiel einer Zange schematisch in drei Bildern dargestellt. Das erste Bild 4a zeigt eine Zange im geöffneten Zustand. Hier findet keine Berührung zwischen Schneidgrat und Schneidbahn statt. Ausgehend von diesem Zustand wird die Zange immer weiter in Richtung eines geschlossenen Zustands bewegt. Die nächste Darstellung 4b zeigt die Position des Kontaktpunktes 45 bzw. des elektrischen Kurzschlusses zu Beginn der Bewegung im gerade geschlossenen Zustand zwischen dem Schneidgrat 41 und der Schneidbahn 40 an deren distalem Ende, also da, wo sich Schneidgrat und Schneidbahn beim Schließen des Effektors zuerst berühren.

Im nächsten Bild 4c ist eine Mittelstellung nach Fortsetzung der Bewegung gezeigt, bei der sich der Kontaktpunkt 46 bzw. elektrische Kurzschluss ungefähr in der Mitte der Länge der Schneidbahn befindet. In Fig. 4d ist der Zustand am Ende der Bewegung dargestellt. Hier befindet sich der Kontaktpunkt bzw. Kurzschluss am proximalen Ende der Effektorelemente bzw. Schneidbahn. Gleichzeitig ist auch die zunehmende Durchbiegung des federelastische Elements 10 dargestellt, die diese hier schematisch dargestellte Verlagerung des elektrischen Kurzschlusses beim Schließen des Effektors von distal nach proximal und beim Öffnen des Effektors von proximal nach distal ermöglicht.

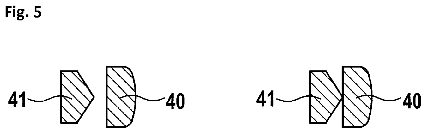

In Fig. 5 sind im Schnitt Schneidgrat und Schneidbahn aus der vorigen Figur dargestellt, wobei links ein beabstandeter Zustand und rechts ein Zustand am Kontaktpunkt dargestellt ist.

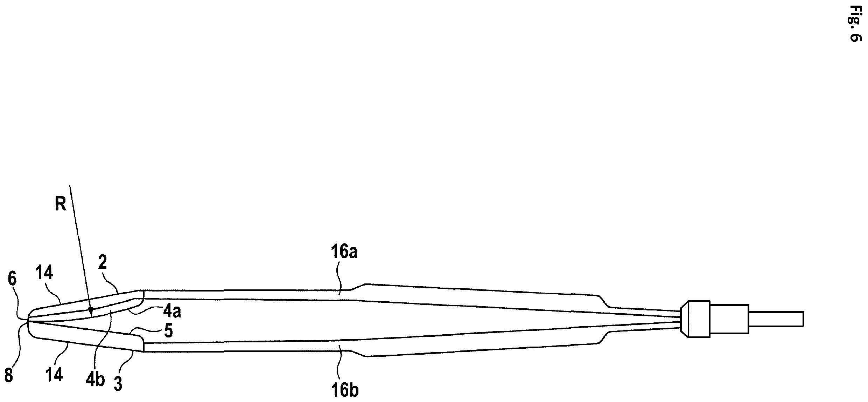

Die Figur 6 zeigt eine Pinzette mit einem Effektor, wie er in diesem Dokument im Detail beschrieben ist.

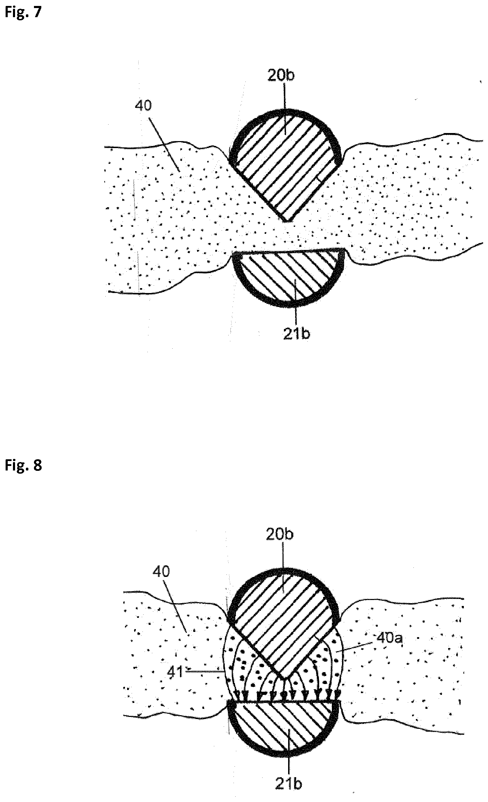

Figur 7 zeigt ein zu durchtrennendes Gewebe 40, das zwischen den Effektorelementen 20b, 21b gegriffen und gequetscht ist. Im Gewebe enthaltene Blutgefäße (nicht dargestellt) sind platt gedrückt.

Figur 8 zeigt einen Zustand, in dem die hochfrequente elektrische Spannung zwischen den Effektorelementen aktiviert ist und elektrische Feldstärken 41 verursacht, deren Amplitude bekanntlich da am größten ist, wo der Abstand zwischen den beiden Effektorelementen am kleinsten ist, also zwischen Schneidgrat und Schneidbahn. Die elektrische Stromdichte im Gewebe ist proportional der elektrischen Feldstärke.

Die Erwärmung des Gewebes 40 zwischen den Effektorelementen 20b, 21b verursacht ab ca. 60°C die thermische Koagulation 40a des Gewebes 40 und ab ca. 100°C die Desikkation des koagulierten Gewebes. Dies geschieht am schnellsten dort, wo die elektrische Stromdichte am größten ist, also zwischen Schneidgrat und Schneidbahn. Die thermische Koagulation und insbesondere Desikkation verursachen eine Versiegelung der platt gedrückten Blutgefäße.

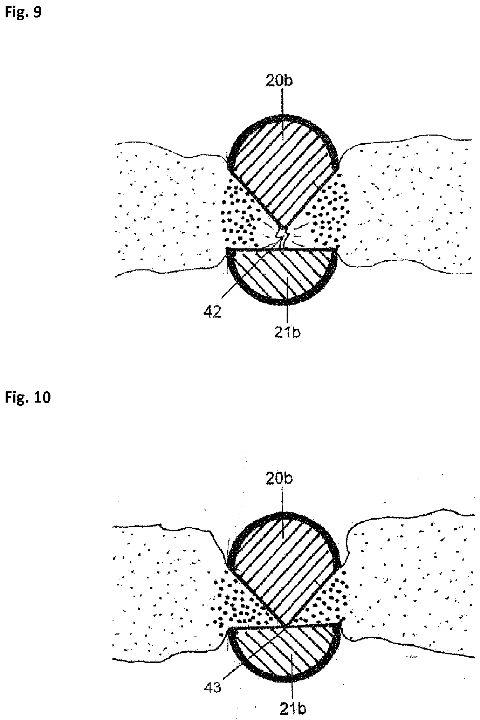

Da die Erwärmung des Gewebes proportional dem Quadrat der elektrischen Stromdichte steigt, wird die für die Bildung elektrischer Lichtbogen 42 erforderliche Dampfphase zwischen Schneidgrat und Schneidfläche schneller erreicht als an anderen Stellen. Dies ist in Figur 9 dargestellt. Die elektrischen Lichtbogen vaporisieren das Gewebe zwischen Schneidgrat und Schneidbahn die HF-chirurgische Gewebetrennung verursacht wird.

Wenn der Schneidgrat des Effektorelements 20b an Stellen, wo infolge Vaporisation kein Gewebe mehr vorhanden ist, die Schneidbahn berührt, entsteht, wie in Figur 10 dargestellt, ein elektrischer Kurzschluss 43 wodurch folglich keine elektrische Feldstärke zwischen den beiden Effektorelementen 20a, 20b vorhanden ist. Sobald nach einer weiteren Bewegung der Effektorelemente an anderer Stelle zwischen distalem und proximalem Ende des Schneidgrats und der Schneidbahn wieder Gewebe zwischen Schneidgrat und Schneidbahn vorhanden ist, wiederholen sich die in den Fig. 7 bis 10 dargestellten Effekte.

## Patentansprüche

1. Bipolarer HF-chirurgischer Effektor (1), umfassend ein erstes Effektor-Element (2) sowie ein zweites Effektor-Element (3), wobei
das erste Effektorelement (2) mit dem zweiten Effektorelement (3) durch ein Gelenk verbunden ist, das eine Bewegung des ersten Effektorelements (2) gegenüber dem zweiten Effektorelement (3) ausschließlich in einer ersten Ebene zulässt, wobei der Effektor (1) zwischen einem geöffneten Zustand und einem geschlossenen Zustand und im geschlossenen Zustand zwischen mehreren Positionen bewegt werden kann,
wobei ein federelastisches Element (x) zwischen dem Gelenk und wenigstens einem der Effektor-Elemente (2, 3) angeordnet ist,
das erste Effektorelement (2) einen elektrisch leitfähigen Schneidgrat (4a) in der ersten Ebene mit wenigstens einer längs des Schneidgrats angeordneten elektrisch leitfähigen Koagulationsflanke (4b, 23a, 23b, 23c) umfasst, das zweite Effektorelement (3) eine gegenüber dem Schneidgrat (4a) angeordnete elektrisch leitfähige Schneidbahn (5) umfasst, und
in einem geöffneten Zustand der Schneidgrat (4a) gegen die Schneidbahn (5) elektrisch isoliert ist und **dadurch gekennzeichnet, dass** in einem geschlossenen Zustand ein elektrischer Kurzschluss zwischen Schneidgrat (4a) und Schneidbahn (5) besteht.

2. Bipolarer HF-chirurgischer Effektor (1) nach Anspruch 1,
**dadurch gekennzeichnet, dass**
der der Schneidgrat (4a) und/oder die Schneidbahn (5) eine konvexe Krümmung aufweist.

3. Bipolarer HF-chirurgischer Effektor (1) nach Anspruch 1 oder 2,
**dadurch gekennzeichnet, dass**
der HF-chirurgische Effektor (1) derart konfiguriert ist, dass im geschlossenen Zustand des Effektors (1) der Schneidgrat (4a) die Schneidbahn (5) in genau einem Punkt berührt, der sich während der Bewegung des Effektors (1) zwischen wenigstens zwei Positionen des geschlossenen Zustands entlang des Schneidgrats (4a) und der Schneidbahn (5) fortbewegt.

4. Bipolarer HF-chirurgischer Effektor (1) nach Anspruch 3,
**dadurch gekennzeichnet, dass**
der HF-chirurgische Effektor (1) derart konfiguriert ist, dass durch eine kontinuierliche Bewegung des Effektors im geschlossenen Zustand eine kontinuierliche Bewegung des Punkts der Berührung bevorzugt ohne Unterbrechung erfolgt und/oder bei einer Fortsetzung der Bewegung des Effektors (1) von einem geöffneten Zustand in einen geschlossenen Zustand, im geschlossenen Zustand eine Bewegung des Punkts der Berührung in proximaler Richtung erfolgt.

5. Bipolarer HF-chirurgischer Effektor (1) nach Anspruch 3 oder 4,
**dadurch gekennzeichnet, dass**
der HF-chirurgische Effektor (1) derart konfiguriert ist, dass an dem Punkt der Berührung ein elektrischer Kurzschluss zwischen dem Schneidgrat (4a) und der Schneidbahn (5) ist.

6. Bipolarer HF-chirurgischer Effektor (1) nach einem der vorhergehenden Ansprüche, **dadurch gekennzeichnet, dass**
der Schneidgrat (4a) an einer Seite eine elektrisch leitfähige Koagulationsflanke (23a, 23b, 23c) oder an zwei Seiten jeweils eine elektrisch leitfähige Koagulationsflanke (23a, 23b, 23c) umfasst.

7. Bipolarer HF-chirurgischer Effektor (1) nach Anspruch 6,
**dadurch gekennzeichnet, dass**
der Schneidgrat (4a) zwei unter einem Flankenwinkel (a) zusammentreffende Flächen umfasst, wobei die zwischen den Flächen entstehende Kante zur Schneidbahn (5) hin orientiert ist und der Flankenwinkel (a) zwischen einer der Flächen und einer Fläche der Schneidbahn (5) wahlweise kleiner 150°, kleiner als 110°, kleiner als 90°, kleiner als 60° ist.

8. Bipolarer HF-chirurgischer Effektor (1) nach einem der vorhergehenden Ansprüche, **dadurch gekennzeichnet, dass**
die Schneidbahn (5) eine Führungsrille (x) für den Schneidgrat (4a) des ersten Effektor-Elements (2) oder einen Schneidgrat umfasst.

9. Bipolarer HF-chirurgischer Effektor (1) nach einem der vorhergehenden Ansprüche, **dadurch gekennzeichnet, dass**
die Oberflächen des Effektors (1) und/oder der Komponenten des Gelenks elektrisch nicht leitfähig sind und/oder mit einem elektrischen Isolationsmaterial beschichtet sind, wobei der Schneidgrat (4a), die Koagulationsflanken (4b, 23a, 23b, 23c) und die Schneidbahn (5, 22a, 22b, 22c) ausgenommen sind und zumindest eine leitfähige Oberfläche umfassen und/oder
dass wenigstens eines von dem Schneidgrat (4a), den Koagulationsflanken (4b, 23a, 23b, 23c) und der Schneidbahn (5, 22a, 22b, 22c) mit einem Material beschichtet sind, das das Ankleben biologischer Gewebe reduziert oder verhindert.

10. Bipolarer HF-chirurgischer Effektor (1) nach einem der vorhergehenden Ansprüche, **dadurch gekennzeichnet, dass**
das federelastische Element mindestens einen elastischen Biegebereich derart umfasst, dass der HF-chirurgische Effektor (1) von einem geöffneten Zustand in mehrere vorgegebene Positionen im geschlossenen Zustand bewegt werden kann, ohne dass eine plastische Verformung eintritt.

11. Bipolarer HF-chirurgischer Effektor (1) nach einem der vorhergehenden Ansprüche, **dadurch gekennzeichnet, dass**
der HF-chirurgische Effektor (1) oder ein mit dem Effektor verbundenes Bauteil einen mechanischen Anschlag aufweist, der die Bewegung des Effektors auf einen Bereich, der kleiner oder gleich dem elastischen Bereich des federelastischen Elements ist, einschränkt.

12. Bipolarer HF-chirurgischer Effektor (1) nach einem der vorhergehenden Ansprüche, **dadurch gekennzeichnet, dass**
das Gelenk, ein Drehgelenk, ein federelastisches Element oder eine Parallelführung umfasst und/oder
der Effektor (1) Bestandteil einer Schere, einer Zange, einer Pinzette oder eines endoskopischen Instruments ist.

13. Bipolarer HF-chirurgischer Effektor (1) nach einem der vorhergehenden Ansprüche, **dadurch gekennzeichnet, dass**
an einem proximalen Ende (12, 13) mindestens eines biegeelastischen Elements (x, 10, 11) und/oder am proximalen Ende (7) des Effektorelements (2) und/oder des Schneidgrats und/oder am proximalen Ende des Effektorelements (3) und/oder der Schneidbahn und/oder am proximalen Ende des Gelenks ein Aktuator vorhanden ist, mit dem der Effektor bewegt und insbesondere geöffnet und geschlossen werden kann.

14. Bipolarer HF-chirurgischer Effektor (1) nach einem der vorhergehenden Ansprüche, **dadurch gekennzeichnet, dass**
das erste Effektorelement (2) mit einem ersten Pol eines bipolaren HF-Chirurgiegenerators verbunden ist und das zweite Effektorelement (3) mit einem zweiten Pol des HF-Chirurgiegenerators verbunden ist.

15. Schere, Zange, Pinzette oder endoskopisches Instrument umfassend einen HF-chirurgischen Effektor (1) nach einem der vorhergehenden Ansprüche.

## Claims

1. Bipolar RF-surgical effector (1), comprising a first effector element (2) and a second effector element (3), wherein the first effector element (2) is connected to the second effector element (3) by a joint that allows movement of the first effector element (2) with respect to the second effector element (3) only in a first plane, wherein the effector (1) can be moved between an open state and a closed state, and in the closed state between several positions,
wherein a resilient element (x) is arranged between the joint and at least one of the effector elements (2, 3),
the first effector element (2) has an electrically conductive cutting ridge (4a) in the first plane with at least one electrically conductive coagulation flank (4b, 23a, 23b, 23c)) arranged along the cutting ridge,
the second effector element (3) comprises an electrically conductive cutting path (5) arranged opposite the cutting ridge (4a), and
in an open state the cutting ridge (4a) is electrically insulated from the cutting path (5), and **characterized in that**
in a closed state there is an electrical short circuit between the cutting ridge (4a) and the cutting path (5).

2. Bipolar RF-surgical effector (1) according to claim 1,
**characterized in that**
the cutting ridge (4a) and/or the cutting path (5) has a convex curvature.

3. Bipolar RF-surgical effector (1) according to claim 1 or 2,
**characterized in that**
the RF-surgical effector (1) is configured such that in a closed state of the effector (1), the cutting ridge (4a) touches the cutting path (5) at exactly one point that moves forward between at least two positions of the closed state along the cutting ridge (4a) and cutting path (5).

4. Bipolar RF surgical effector (1) according to claim 3,
**characterized in that**
the RF surgical effector (1) is configured such that by continuous movement of the effector in the closed state a continuous movement of the point of contact preferably without interruption is performed and/or when the movement of the effector (1) continues from an open state into a closed state, in the closed state there is a movement of the point of contact in proximal direction.

5. Bipolar RF surgical effector (1) according to claim 3 or 4,
**characterized in that**
the RF-surgical effector (1) is configured such that at the point of contact there is an electrical short circuit between the cutting ridge (4a) and the cutting path (5).

6. Bipolar RF-surgical effector (1) according to one of the preceding claims,
**characterized in that**
the cutting ridge (4a) has an electrically conductive coagulation flank (23a, 23b, 23c) on one side, or has an electrically conductive coagulation flank (23a, 23b, 23c), on each of two sides respectively.

7. Bipolar RF surgical effector (1) according to claim 6,
**characterized in that**
the cutting ridge (4a) comprises two surfaces meeting at a flank angle (α), wherein the edge arising between the surfaces is oriented towards the cutting path (5) and the flank angle (α) between one of the surfaces and a surface of the cutting path (5) optionally is smaller than 150°, smaller than 110°, smaller than 90°, smaller than 60°.

8. Bipolar RF-surgical effector (1) according to one of the preceding claims,
**characterized in that**
the cutting path (5) comprises a guiding groove (x) for the cutting ridge (4a) of the first effector element (2) or a cutting ridge.

9. Bipolar RF-surgical effector (1) according to one of the preceding claims,
**characterized in that**
the surfaces of the effector (1) and/or of the components of the joint are electrically non-conductive and/or are coated with an electrical insulating material,
wherein the cutting ridge (4a), the coagulation flanks (4b, 23a, 23b, 23c) and the cutting path (5, 22a, 22b, 22c) are excluded and comprise at least a conductive surface
and/or
that at least one of the cutting ridge (4a), the coagulation flanks (4b, 23a, 23b, 23c) and the cutting path (5, 22a, 22b, 22c) are coated with a material that reduces or prevents the adhesion of biological tissue.

10. Bipolar RF-surgical effector (1) according to one of the preceding claims,
**characterized in that**
the resilient element comprises at least one elastic bending area such that the RF-surgical effector (1) can be moved from an open state into several predetermined positions in the closed state without any plastic deformation occurring.

11. Bipolar RF-surgical effector (1) according to one of the preceding claims,
**characterized in that**
the RF-surgical effector (1) or a component connected to the effector has a mechanical stop which limits the movement of the effector to an area that is less than or equal to the elastic range of the resilient element.

12. Bipolar RF surgical effector (1) according to one of the preceding claims,
**characterized in that**
the joint comprises a swivel joint, a resilient element or a parallel guide and/ or
the effector (1) is part of scissors, a forceps, a tweezers or an endoscopic instrument.

13. Bipolar RF-surgical effector (1) according to one of the preceding claims,
**characterized in that**
at a proximal end (12, 13) of at least one flexural element (x, 10, 11) and/or at the proximal end (7) of the effector element (2) and/or of the cutting ridge and/or at the proximal end of the effector element (3) and/or of the cutting path and/or at the proximal end of the joint there is an actuator with which the effector can be moved and in particular can be opened and closed.

14. Bipolar RF surgical effector (1) according to one of the preceding claims,
**characterized in that**
the first effector element (2) is connected to a first pole of a bipolar RF-surgical generator and the second effector element (3) is connected to a second pole of the RF-surgical generator.

15. Scissors, forceps, tweezers or endoscopic instrument comprising an RF-surgical effector (1) according to one of the preceding claims.

## Revendications

1. Effecteur de chirurgie HF bipolaire (1) comprenant un premier élément d'effecteur (2) et un deuxième élément d'effecteur (3), dans lequel le premier élément d'effecteur (2) est relié au deuxième élément d'effecteur (3) par une articulation qui permet un mouvement du premier élément d'effecteur (2) par rapport au deuxième élément d'effecteur (3) uniquement dans un premier plan, l'effecteur (1) pouvant être déplacé entre un état ouvert et un état fermé et, dans l'état fermé, entre plusieurs positions,
dans lequel un élément formant un ressort élastique (x) est disposé entre l'articulation et au moins un des éléments d'effecteur (2, 3),
le premier élément d'effecteur (2) comprend une arête de coupe (4a) conductrice électrique avec au moins un flanc de coagulation (4b, 23a, 23b, 23c) conducteur électrique disposé le long de l'arête coupante, le deuxième élément effecteur (3) comprend une piste de coupe (5) conductrice électrique disposée en face de l'arête de coupe (4a) et l'arête de coupe (4a) est isolée électriquement de la piste de coupe (5) dans l'état ouvert,
**caractérisé en ce que**, dans un état fermé, un court-circuit électrique s'établit entre l'arête de coupe (4a) et la piste de coupe (5).

2. Effecteur de chirurgie HF bipolaire (1) selon la revendication 1, **caractérisé en ce que** l'arête de coupe (4a) et/ou la piste de coupe (5) présentent une courbure convexe.

3. Effecteur de chirurgie HF bipolaire (1) selon la revendication 1 ou 2, **caractérisé en ce que** l'effecteur de chirurgie HF (1) est configuré de telle manière que, quand l'effecteur (1) est dans l'état fermé, l'arête de coupe (4a) touche la piste de coupe (5) en exactement un point qui se déplace, pendant le mouvement de l'effecteur (1) entre au moins deux positions de l'état fermé, le long de l'arête de coupe (4a) et de la piste de coupe (5).

4. Effecteur de chirurgie HF bipolaire (1) selon la revendication 3, **caractérisé en ce que** l'effecteur de chirurgie HF (1) est configuré de telle manière qu'un mouvement continu de l'effecteur dans l'état fermé entraîne un déplacement continu du point de contact, de préférence sans interruption, et/ou si le mouvement de l'effecteur (1) se poursuit d'un état ouvert à un état fermé, le point de contact se déplace dans la direction proximale.

5. Effecteur de chirurgie HF bipolaire (1) selon la revendication 3 ou 4, **caractérisé en ce que** l'effecteur de chirurgie HF (1) est configuré de telle manière qu'un court-circuit se produise entre l'arête de coupe (4a) et la piste de coupe (5) au point où elles se touchent.

6. Effecteur de chirurgie HF bipolaire (1) selon l'une des revendications précédentes, **caractérisé en ce que** l'arête de coupe (4a) comprend un flanc de coagulation électriquement conducteur (23a, 23b, 23c) sur un côté ou un flanc de coagulation électriquement conducteur (23a, 23b, 23c) sur deux côtés.

7. Effecteur de chirurgie HF bipolaire (1) selon la revendication 6, **caractérisé en ce que** l'arête de coupe (4a) comprend deux surfaces qui se rejoignent en formant un angle de flancs (α), l'arête formée entre les surfaces étant orientée vers la piste de coupe (5) et l'angle de flancs (α) entre une des surfaces et une surface de la piste de coupe (5) est, au choix, plus petit que 150°, plus petit que 110°, plus petit que 90°, plus petit que 60°.

8. Effecteur de chirurgie HF bipolaire (1) selon l'une des revendications précédentes, **caractérisé en ce que** la piste de coupe (5) comprend une rainure de guidage (x) pour l'arête de coupe (4a) du premier élément d'effecteur (2) ou une arête de coupe.

9. Effecteur de chirurgie HF bipolaire (1) selon l'une des revendications précédentes, **caractérisé en ce que** les surfaces de l'effecteur (1) et/ou des composants de l'articulation ne sont pas conductrices électriques et/ou sont revêtues d'un matériau isolant électrique, l'arête de coupe (4a), les flancs de coagulation (4b, 23a, 23b, 23c) et la piste de coupe (5, 22a, 22b, 22c) sont exclus et comprennent au moins une surface conductrice et/ou **en ce qu'**au moins un élément parmi l'arête de coupe (4a), les flancs de coagulation (4b, 23a, 23b, 23c) et la piste de coupe (5, 22a, 22b, 22c) sont revêtus d'un matériau qui réduit ou empêche l'adhérence de tissus biologiques.

10. Effecteur de chirurgie HF bipolaire (1) selon l'une des revendications précédentes, **caractérisé en ce que** l'élément formant un ressort élastique comprend au moins une zone de flexion élastique, de telle manière que l'effecteur de chirurgie HF (1) puisse être déplacé d'un état ouvert à plusieurs positions prédéterminées dans l'état fermé sans qu'il se produise de déformation plastique.

11. Effecteur de chirurgie HF bipolaire (1) selon l'une des revendications précédentes, **caractérisé en ce que** l'effecteur de chirurgie HF (1) ou un composant relié à l'effecteur présente une butée mécanique qui limite le mouvement de l'effecteur à une course inférieure ou égale à la course élastique de l'élément formant un ressort élastique.

12. Effecteur de chirurgie HF bipolaire (1) selon l'une des revendications précédentes, **caractérisé en ce que** l'articulation comprend une articulation pivotante, un élément formant un ressort élastique ou un guidage parallèle et/ou
l'effecteur (1) fait partie de ciseaux, d'un forceps, d'une pince ou d'un instrument endoscopique.

13. Effecteur de chirurgie HF bipolaire (1) selon l'une des revendications précédentes, **caractérisé en ce qu'**il comporte à une extrémité proximale (12, 13) d'au moins un élément élastique en flexion (x, 10, 11) et/ou à l'extrémité proximale (7) de l'élément effecteur (2) et/ou de l'arête de coupe et/ou à l'extrémité proximale de l'élément effecteur (3) et/ou de la piste de coupe et/ou à l'extrémité proximale de l'articulation un vérin avec lequel l'effecteur peut être déplacé, en particulier ouvert et fermé.

14. Effecteur de chirurgie HF bipolaire (1) selon l'une des revendications précédentes, **caractérisé en ce que** le premier élément d'effecteur (2) est relié à un premier pôle d'un générateur de chirurgie HF bipolaire et le deuxième élément effecteur (3) à un deuxième pôle du générateur de chirurgie HF bipolaire.

15. Ciseaux, forceps, pince ou instrument endoscopique comprenant un effecteur de chirurgie HF (1) selon l'une des revendications précédentes.
